# EUROPEAN PATENT SPECIFICATION

(11) **EP 0 364 454 B1**
(45) Date of publication and mention of the grant of the patent: **30.06.1993**
(21) Application number: 88902965.8
(22) Date of filing: 09.03.1988
(51) Int. Cl.: A61F 13/15, A61F 13/82

(54) **DEVICE FOR THE SUPPORT OF AN ABSORBENT ARTICLE**
HALTER FÜR SAUGEINLAGEN
DISPOSITIF DE SOUTIEN D'UN ARTICLE ABSORBANT

(30) Priority: 24.03.1987 SE 8701208
(43) Date of publication of application: 25.04.1990
(73) Proprietor: Mölnlycke AB, 405 03 Göteborg (SE)
(72) Inventor: YGGE, Birgitta, S-413 24 Göteborg (SE); VIGMO, Terje, S-435 41 Mölnlycke, (SE)
(74) Representative: Kierkegaard, Lars-Olov
(86) International application number: SE8800116
(87) International publication number: WO8807335

(56) References cited:
- US-A- 3 441 025
- US-A- 4 560 381

## Description

The present invention relates to a sanitary garment comprising an absorbent article, such as an incontinence protector, a diaper or the like, a waist belt for supporting the article and fastening means for affixing the article to the waist belt.

Numerous types of devices for supporting absorbent aticles are previously known. For example, elastic textile pants are often utilized to hold diapers or the like fixed in position during use. An alternative to such pants is the so-called all-in-one diaper which is composed of a plastic backing forming together with a diaper an integral unit. In its unused state, the most common type of all-in-one diapers is principally shaped as a pair of underpants with open side seams which are joined together with adhesive tape when using the diaper so as to make it seal like a pant around the user's abdomen.

By being both easily handled and readily replaceable, the all-in-one diapers are certainly most useful in practice. Among adults suffering from incontinence however, the wide range of individual variations as to degree of incontinence and to bodily shape and size makes it almost impossible for economic and manufacturing reasons to satisfy all demands with only a limited number of diaper variants available.

Therefore, a diaper fixed in position with the aid of a separate pant would be more useful for incontinent adults. By being able to chose the appropriate size of pant independent of the type of diaper required with regard to degree of incontinence, there is provided for the user a large selection of combination possibilities from only a restricted number of pant and diaper sizes.

There is however a drawback associated with complete diaper pants in that they are difficult to put on and replace on users of a specific category such as those incontinent, institutionalized patients who are heavy immobile and incapable of standing on their legs, and bed-ridden or contractured patients. The use of complete pants, which have to be wrenched over the user's legs to be properly applied, naturally complicates diaper change making it a time-consuming procedure for the nursing staff and awkward for the incontinent patient.

The application of pants which have to be threaded on over the feet may even cause trouble to disabled persons or those with coordination problems but otherwise capable of managing on their own at home.

A sanitary garment of the kind set forth in the preamble of Claim 1 is known from US-A- 3 441 025. However, the fastening means for attaching the absorbent article to the waist belt consists of a reusable cover of flexible and moisture impervious material, said cover being affixed to the waist belt with the help of adjustable straps secured to the waist belt having snap fasteners for the attachment to the cover. The application of such a device is rather complicated, comprising the steps of placing an absorbent pad in the cover, adjusting the straps in accordance with the size of the user and fastening the straps to the cover and such a device is therefore not convenient to use for the abovementioned type of users. Moreover, it is not unlikely that the cover is soiled at the time for replacing the absorbent pad and therefore also has to be changed, which would lead to a lot of laundring.

With the present invention however there is accomplished a sanitary garment of the kind set forth in Claim 1 and which eliminates the problem associated with previously known items of this type.

Preferred embodiments of the invention are stated in the subclaims.

The sanitary garment including a waist strap or belt which is readily connectible with a diaper affords the possibility of selecting waist belt and diaper independently of one another. Moreover, application of the waist belt, especially if openable, onto the user's body is a most simple procedure. An additional advantage is gained in that even those with restricted ability of turning or bending down may easily manage to change on their own a diaper attached to a waist belt according to the present invention. The manner of performing such a diaper change will be described in the following.

In contrast to complete pants, a waist belt according to the present invention will generally remain unsoiled when worn and may be used several times before it needs washing. Of course, waist belts intended to be discarded when soiled are also conceivable.

A sanitary garment according to the invention will be described in more detail below with reference to the exemplary embodiments illustrated in the accompanying drawings, of which
Fig. 1 is a plan view of a diaper provided with the inventive attachment means; and
Figs. 2,3 and 4 show different embodiments of a waist belt for use in combination with the diaper illustrated in Fig. 1;
The diaper shown in Fig. 1 is of a conventional type, comprising a liquid permeable inner layer 2, a liquid impermeable outer layer 3, and enclosed therebetween an absorbent body 4.

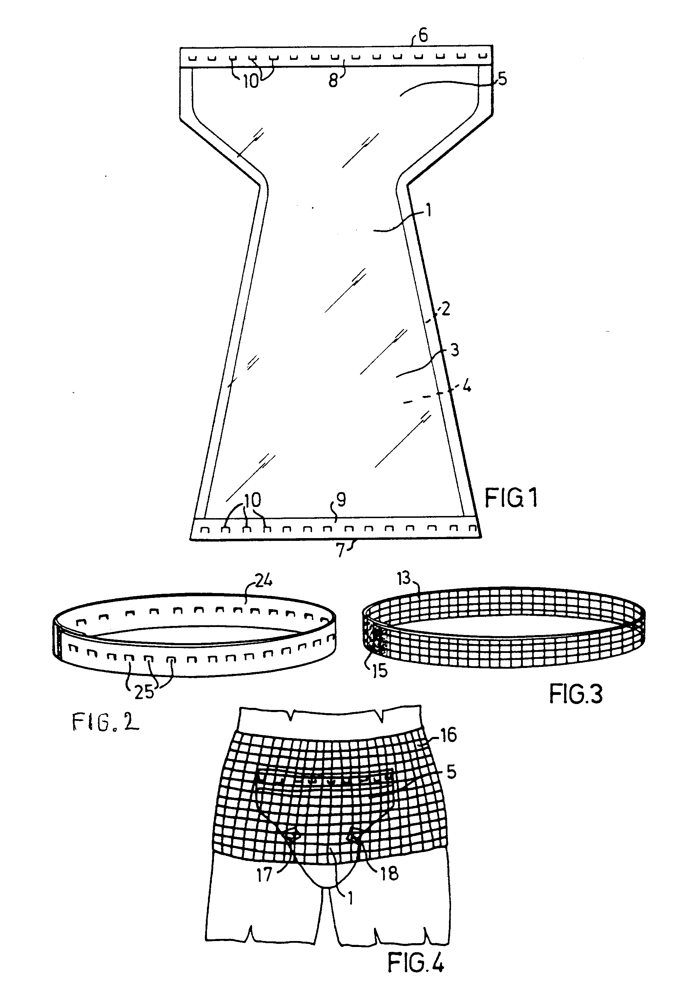

The diaper 1 has the shape of a T and is intended to be worn with the cross beam 5 of the T applied to fit snugly over the user's belly (see Fig. 4).

Bands 8, 9 provided with hooks and loops elements of Velcro tape type are disposed at either transverse end 6, 7 of the diaper, which bands 8,9 can be made of plastics or textile. The bands 8, 9 shown in Figs. 1 and 3 are plastic bands provided with Velcro tape elements 10.

The diaper 1 shown in Fig. 1 is intended for use together with a waist belt having cooperating Velcro tape elements for engagement with the elements 10 provided on the bands 8,9. Such waist belts are shown in Figs. 2 and 3. The waist belt 24 of Fig. 2 is made of plastics or textile whereas the waist belt 13 of Fig. 3 is made in the form of an elastic netting. In order to facilitate application, the waist belts can be opened and closed with the aid of fastening means 14, 15 which in the exemplary embodiment also are Velcro type closures.

A waist belt 16 can be made wide enough, as in Fig. 4, to overlap the diaper 1 during use for holding it tightly pressed against the wearer's body. In this manner the diaper will remain more securely in position reducing thereby the risk of leakage past the diaper edges. With a wider waist belt 16, however, additional points of attachment 17,18 may be required between the diaper and the waist belt. In the exemplary embodiment, such points of attachment 17,18 are arranged at the groin region of the wearer.

To advantage, the application of a diaper and the inventive waist belt onto a user's body takes place in the following manner: The waist belt is applied around the user's waist whereafter the rear end of the diaper is affixed to the forward portion of the waist belt. The belt is then rotated around the user's waist until the diaper has moved halfway around and is left suspended behind the user's back.

The forward free end of the diaper is finally brought up from between the user's legs and is attached to the waist belt across the user's belly. Since all twisting or bending movements of the body can be avoided throughout the procedure, even persons with disabled backs and legs would be able to put on and change diapers without help.

## Claims

1. A sanitary garment comprising an absorbent article (1), such as an incontinence protector, a diaper or the like, a waist belt (13,16,24) for supporting the article and fastening means for affixing the article to the waist belt, **characterized** in that the fastening means consist of coacting hook and loop fabric tape elements (10,25) of the Velcro tape type secured to the article (1) and the waist belt (13,16,24), the waist belt or the article comprising several rows of hook or loop members, said rows being oriented in parallel with the transverse direction of the article in its applied state.

2. A sanitary garment according to Claim 1, **characterized** in that bands (8,9) provided with hook or loop members (10) are disposed at either transverse end (6,7) of the article and extend the whole width of the respective end (6,7).

3. A sanitary garment according to Claim 1 or 2, **characterized** in that the waist belt (13,24) comprises reopenable fastenings elements (14,15).

## Patentansprüche

1. Halter für Saugeinlagen (1), wie zum Beispiel für einen Inkontinenz-Schutz, eine Windel oder dergleichen, mit einem Gürtel (13, 16, 24), der den Artikel hält und mit Befestigungsmitteln zum Befestigen des Artikels am Gürtel, dadurch **gekenn zeichnet,** daß die Befestigungsmittel aus Bandelementen mit zusammenwirkenden Haken und Ösen (10, 25) nach Art von Klettbändern bestehen, die am Artikel (1) und am Gürtel (13, 16, 24) befestigt sind, wobei der Gürtel oder der Artikel mehrere Reihen von Haken- oder ösenteilen aufweist und wobei die Reihen zu der Querrichtung des Artikels in seinem angezogenen Zustand parallel verlaufen.

2. Halter für Saugeinlagen nach Anspruch 1, dadurch gekennzeichnet, daß Bänder (8, 9), die mit den Haken- oder Ösenteilen (10) versehen sind, an beiden, quer verlaufenden Enden (6, 7) des Artikels (1) angeordnet sind und sich über die gesamte Breite des jeweiligen Endes (6, 7) erstrecken.

3. Halter für Saugeinlagen nach einem der Ansprüche 1 oder 2, dadurch gekennzeichnet, daß der Gürtel (13, 24) zu öffnende Befestigungselemente (14, 15) aufweist.

## Revendications

1. Vêtement hygiénique comprenant un article absorbant (1), comme une protection pour incontinent, une couche ou un autre article similaire, une ceinture (13, 16. 24) pour soutenir l'article et des moyens de fixation pour attacher l'article à la ceinture, caractérisé en ce que les moyens de fixation sont des éléments (10, 25) de bande de tissu à boucles et crochets coopérants, du type bande Velcro, qui sont fixés à l'article (1) et à la ceinture (13, 16, 24), la ceinture ou l'article comprenant plusieurs rangées d'éléments à boucles ou à crochets, ces dites rangées étant parallèles à la direction transversale de l'article lorsqu'il est mis en place.

2. Vêtement hygiénique selon la revendication 1, caractérisé en ce que les bandes (8, 9) pourvues d'éléments (10) à boucles ou crochets sont placées aux deux extrémités transversales (6, 7) de l'article et s'étendent sur toute la largeur de l'extrémité respective (6, 7).

3. Vêtement hygiénique selon la revendication 1 ou 2. caractérisé en ce que la ceinture (13, 24) comporte des éléments (14, 15) de fixation ouvrables.
